# EUROPEAN PATENT APPLICATION

(11) **EP 3 943 587 A1**
(43) Date of publication of application: **26.01.2022**
(21) Application number: 20773518.4
(22) Date of filing: 17.03.2020
(51) Int. Cl.: C12M 1/00, C12M 3/00

(54) **CULTURE SYSTEM, CULTURE DEVICE, AND MULTI-LAYER CULTURE VESSEL MANIPULATION DEVICE**

(30) Priority: 20.03.2019 JP 2019052299; 13.02.2020 JP 2020022171
(71) Applicant: Shikoku Instrumentation Co., Ltd., Kagawa 764-8502 (JP); Taisei Corporation, Tokyo 163-0606 (JP)
(72) Inventor: KATAOKA, Tadashi, Nakatado-gun, Kagawa 764-8502 (JP); NAKANISHI, Takafumi, Nakatado-gun, Kagawa 764-8502 (JP); MORI, Toshiaki, Nakatado-gun, Kagawa 764-8502 (JP); TANAKA, Toshiaki, Tokyo 163-0606 (JP); MATSUMURA, Yoshiyuki, Tokyo 163-0606 (JP)
(74) Representative: Gulde & Partner
(86) International application number: PCT/JP2020/011606
(87) International publication number: WO 2020/189655

(57) **Abstract**

Problem: To provide a culture system and a culture device each of which can minimize the burden on workers and can effectively prevent the effect of temperature changes on culture by performing manipulation of a multilayer culture vessel and the culture in sequence in the same space. Solution: The culture system comprises a housing 11 with an internal space S in which a multilayer culture vessel 12 including a plurality of trays 121 therein is placed, and a manipulator 14 manipulating the multilayer culture vessel 12 while the multilayer culture vessel 12 is kept in a state placed within the internal space S. The multilayer culture vessel 12 is communicated with a liquid supply tube 13 such that a fluid material can be introduced into the multilayer culture vessel 12 from an outside of the housing 11 via the liquid supply tube 13, or that a fluid can be discharged from the multilayer culture vessel 12 to the outside of the housing 11 via the liquid supply tube 13.

## Description

### Technical Field

The present invention relates to a culture system, a culture method, and a multilayer culture vessel manipulation device each of which enables cells to be cultured with a multilayer culture vessel including a plurality of trays therein and can perform the cell culture and manipulation of the multilayer culture vessel in the same space.

### Background Art

Aiming to efficiently culture a large amount of cells in a small space, there has hitherto been known a technique of culturing the cells with a multilayer culture vessel including a plurality of trays stacked therein (see, for example, Patent Document 1). There has also been known a device in which handling manipulation of holding and rotating the multilayer culture vessel at the time of introducing a culture solution and so on into the multilayer culture vessel or recovering them from the multilayer culture vessel is performed to reduce the burden on workers when the cells are cultured with the multilayer culture vessel as described above (see Patent Document 2).

### Citation List

### Patent Documents

Patent Document 1: Japanese Patent Laid-Open Publication No. 2016-103984
Patent Document 2: Japanese Patent Laid-Open Publication No. 2015-505472

### Summary of Invention

### Technical Problem

In the case of culturing the cells with the multilayer culture vessel, the culture is performed through a series of steps of manipulating the multilayer culture vessel to fill a culture solution into the multilayer culture vessel, culturing the cells after placing the multilayer culture vessel into an incubator, manipulating the multilayer culture vessel to discharge the culture solution from the multilayer culture vessel after the cell culture, manipulating the multilayer culture vessel to fill a releasing solution of trypsin, etc. into the multilayer culture vessel for releasing of the cells, swinging the multilayer culture vessel to promote the releasing of the cells, and manipulating the multilayer culture vessel to recover the releasing solution of trypsin, etc., including the cells, from the multilayer culture vessel after the releasing of the cells.

Because the above-mentioned series of operations imposes a great burden on workers, a device for performing the handling manipulation of the multilayer culture vessel is proposed as disclosed in the above-cited Patent Document 2. However, the handling manipulation needs a space with a certain large size and is performed outside the incubator in many cases from the viewpoint of cost. If the multilayer culture vessel is taken out to the outside of the incubator when the culture solution and the releasing solution of trypsin, etc. are filled into and discharged from the multilayer culture vessel, when the cells in the multilayer culture vessel are observed with a microscope, or when the releasing solution of trypsin, etc., including the cells, is recovered from the multilayer culture vessel, there is a risk of affecting a temperature fall on the cell culture or causing contamination due to, for example, operations of switching over liquid supply tubes connected to the multilayer culture vessel. Accordingly, there has been demanded a device capable of performing the manipulation of in the multilayer culture vessel and the cell culture in the same space while the multilayer culture vessel is kept in a state held within the incubator.

An object of the present invention is to provide a culture system, a culture device, and a multilayer culture vessel manipulation device each of which can reduce the burden on workers, can prevent the adverse effect of temperature changes, and can effectively prevent the contamination by performing manipulation of a multilayer culture vessel, cell observation, and cell culture in sequence in the same space.

### Solution to Problem

The present invention provides a culture system comprising a housing with an internal space in which a multilayer culture vessel including a plurality of trays therein is placed, and a manipulator manipulating the multilayer culture vessel while the multilayer culture vessel is kept in a state placed within the internal space, wherein the multilayer culture vessel is communicated with a liquid supply tube such that a fluid material can be introduced into the multilayer culture vessel from an outside of the housing via the liquid supply tube, or that a fluid can be discharged from the multilayer culture vessel to the outside of the housing via the liquid supply tube.

In the culture system described above, the manipulator may include a rotation unit rotating or swinging the multilayer culture vessel.

In the culture system described above, the manipulator may include an opening/closing unit opening and closing a communication path between the liquid supply tube and the multilayer culture vessel.

The culture system described above may further comprise an air filter between an inside and an outside of the multilayer culture vessel, and the manipulator may include an opening/closing unit opening and closing a communication path between the air filter and the multilayer culture vessel.

In the culture system described above, the housing may include an insertion portion at which the liquid supply tube penetrates through the housing, and via both the insertion portion and the liquid supply tube, the fluid material may be introduced into the multilayer culture vessel from the outside of the housing, or the fluid may be discharged from the multilayer culture vessel to the outside of the housing.

In the culture system described above, the liquid supply tube may be communicated with a plurality of containers or devices present outside the housing in a switchable manner.

In the culture system described above, a plurality of separate liquid supply tubes in communication with the plurality of containers in a one-to-one relation may be communicated with a common liquid supply tube that is communicated with the multilayer culture vessel, valves may be disposed on the separate liquid supply tubes or at junctions between the separate liquid supply tubes and the common liquid supply tube, and communication states between the separate liquid supply tubes and the common liquid supply tube may be controllable with control of the valves.

The culture system described above may further comprise an observation device disposed under the multilayer culture vessel and taking an image of an inside of the multilayer culture vessel.

The present invention further provides a culture device comprising a housing with an internal space in which a multilayer culture vessel including a plurality of trays therein is placed, and a manipulator manipulating the multilayer culture vessel while the multilayer culture vessel is kept in a state placed within the internal space, wherein the multilayer culture vessel is communicated with a liquid supply tube such that a fluid material can be introduced into the multilayer culture vessel from an outside of the housing via the liquid supply tube, or that a fluid can be discharged from the multilayer culture vessel to the outside of the housing via the liquid supply tube.

In the culture device described above, the manipulator may include a rotation unit rotating or swinging the multilayer culture vessel.

In the culture device described above, the manipulator may include an opening/closing unit opening and closing a communication path between the liquid supply tube and the multilayer culture vessel.

The present invention still further provides a multilayer culture vessel manipulation device placed within a housing and including a manipulator that manipulates a multilayer culture vessel including a plurality of trays therein, wherein the multilayer culture vessel is communicated with the liquid supply tube inside the housing, the manipulator is able to manipulate the multilayer culture vessel while the multilayer culture vessel is kept in a state placed within the housing, and with the manipulator manipulating the multilayer culture vessel, a fluid material can be introduced into the multilayer culture vessel from an outside of the housing via the liquid supply tube, or a fluid can be discharged from the multilayer culture vessel to the outside of the housing via the liquid supply tube.

In the multilayer culture vessel manipulation device described above, the manipulator may include a rotation unit rotating or swinging the multilayer culture vessel.

In the multilayer culture vessel manipulation device described above, the rotation unit may have a first rotation axis and a second rotation axis each serving as a rotation axis about which the multilayer culture vessel is rotated or swung, and the rotation unit may be constituted such that the first rotation axis and the second rotation axis are each a rotation axis passing the multilayer culture vessel.

In the multilayer culture vessel manipulation device described above, the manipulator may include an opening/closing unit opening and closing a communication path between the liquid supply tube and the multilayer culture vessel.

The multilayer culture vessel manipulation device described above may further comprise a liquid level sensor detecting a liquid level of the fluid material in the multilayer culture vessel when the fluid material is introduced into the multilayer culture vessel from the outside of the housing via the liquid supply tube.

In the multilayer culture vessel manipulation device described above, the liquid level sensor may include a first liquid level sensor detecting whether the fluid material has reached a first water level indicating that a predetermined amount of the fluid material has been introduced into the multilayer culture vessel, or a second liquid level sensor detecting whether the fluid material has reached a second water level being lower than the first water level and indicating that the fluid material has been introduced in an amount close to the predetermined amount.

### Advantageous Effects of Invention

According to the present invention, since the manipulation of the multilayer culture vessel and the cell culture can be performed in sequence in the same space, it is possible to reduce the burden on workers, to prevent the adverse effect of temperature changes, and to effectively prevent the contamination.

### Brief Description of the Drawings

[Fig. 1] Fig. 1 is a block diagram illustrating a configuration of a culture system according to a first embodiment.
[Fig. 2] Fig. 2 is a perspective view of a culture device according to the first embodiment.
[Fig. 3] Fig. 3 is an explanatory view illustrating a multilayer culture vessel according to the first embodiment.
[Fig. 4] Fig. 4 is an explanatory view illustrating a manner of distributing a fluid material to individual trays in the multilayer culture vessel.
[Fig. 5] Fig. 5 is an explanatory view illustrating an example of structures of an opening/closing unit and a clamp.
[Fig. 6] Fig. 6 is a flowchart illustrating a cell culture method according to the first embodiment.
[Fig. 7] Fig. 7 is an explanatory view illustrating an operation of rotating the multilayer culture vessel according to the first embodiment.
[Fig. 8] Fig. 8 is a perspective view of a culture system according to a second embodiment.
[Fig. 9] Fig. 9 is a perspective view illustrating a structure inside a housing of the culture system according to the second embodiment.
[Fig. 10] Fig. 10 is a perspective view illustrating the structure inside the housing of the culture system according to the second embodiment when viewed from below.

### Description of Embodiments

Embodiments of a culture system, a culture device, and a multilayer culture vessel manipulation device according to the present invention will be described below with reference to the drawings.

### <First Embodiment

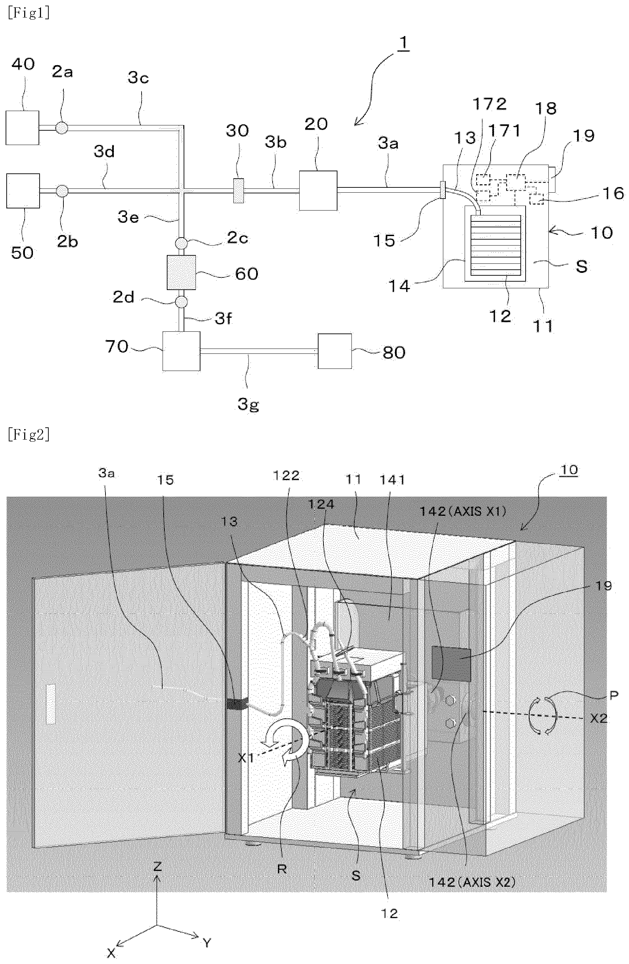

Fig. 1 is a block diagram illustrating a configuration of a culture system 1 according to a first embodiment, and Fig. 2 is a perspective view of a culture device 10 according to the first embodiment. The culture system 1 according to the first embodiment includes, as illustrated in Fig. 1, the culture device 10 accommodating a multilayer culture vessel 12, liquid supply pumps 20 and 70 each supplying a culture solution or a releasing solution of trypsin, etc., a flowmeter 30 measuring a flow rate of the culture solution or the releasing solution of trypsin, etc., containers 40, 50 and 80 from and to each of which the culture solution or the releasing solution of trypsin, etc. is supplied or recovered, and a centrifugal separator 60.

Furthermore, in the culture system 1, as illustrated in Fig. 1, the individual components are communicated via liquid supply tubes 3a to 3g, and flow paths (formed by the liquid supply tubes 3a to 3g) through each of which the culture solution or the releasing solution of trypsin, etc. is supplied are switchable by valves 2a to 2d. The valves 2a to 2d may be operated to switch over manually by a worker or automatically by a mechanical device.

The culture device 10 includes, as illustrated in Figs. 1 and 2, a housing 11, the multilayer culture vessel 12, a liquid supply tube 13, a manipulator 14, an insertion portion 15, a temperature adjustment unit 16, a gas concentration adjustment unit 171, a pH adjustment unit 172, a control unit 18, and an input unit 19. As illustrated in Fig. 2, the culture device 10 accommodates the multilayer culture vessel 12 in an internal space S of the housing 11. The housing 11 can be opened and closed, but it is used in an enclosed state when cell culture is performed. Although there are no special limitations on a size of the internal space S of the housing 11, the internal space S is designed as appropriate depending on the size of the multilayer culture vessel 12 such that the multilayer culture vessel 12 can be rotated or swung by the manipulator 14 as described later.

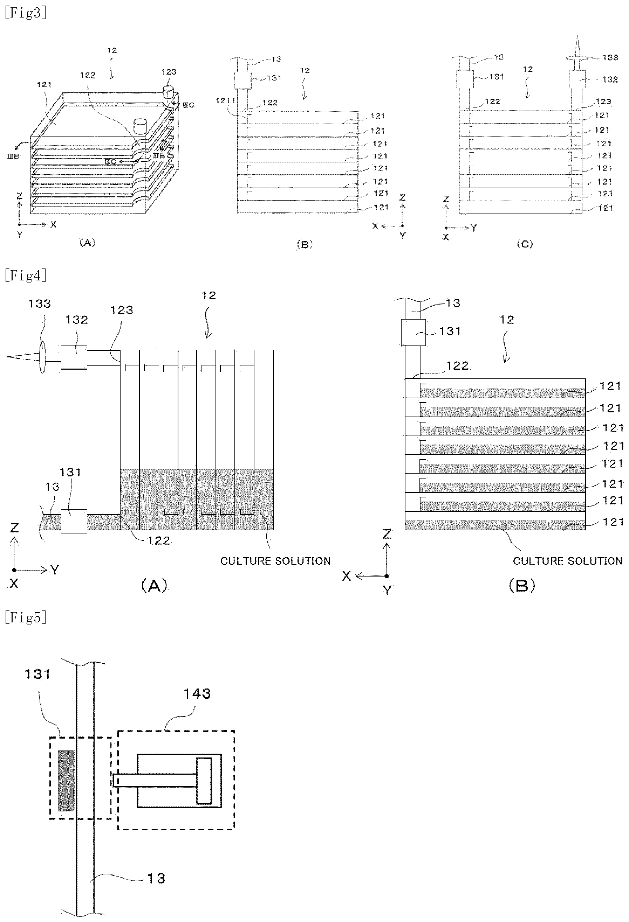

The multilayer culture vessel 12 according to the first embodiment is now described. Fig. 3(A) is an explanatory perspective view illustrating the multilayer culture vessel 12 according to the first embodiment and is a schematic view when looking at the multilayer culture vessel 12 in a direction from the left side toward the right side in Fig. 2. Fig. 3(B) is a schematic sectional view of the multilayer culture vessel 12 taken along IIIB - IIIB in Fig. 3(A), and Fig. 3(C) is a schematic sectional view of the multilayer culture vessel 12 taken along IIIC - IIIC in Fig. 3(A). For convenience of explanation, walls 1211 of trays 121 are omitted in Fig. 3(A). To efficiently culture cells, as illustrated in Figs. 3(A) to 3(C), the multilayer culture vessel 12 is constituted by stacking a plurality of the trays 121 one above another. In each of the trays 121 in the multilayer culture vessel 12, as illustrated in Fig. 3(A), holes are formed at two of four corners. Thus, in the multilayer culture vessel 12, as illustrated in Fig. 3(A), the plurality of the trays 121 are spatially communicated with one another, with the liquid supply tube 13 via a connecting portion 122, and with an air filter 133 via a connecting portion 123, respectively. Furthermore, a clamp 131 is disposed in the liquid supply tube 13 at a position close to the connecting portion 122 of the multilayer culture vessel 12. By opening the clamp 131, via the liquid supply tube 13, the culture solution or the releasing solution of trypsin, etc. can be introduced into the multilayer culture vessel 12, or the culture solution or the releasing solution of trypsin, etc. can be discharged from the multilayer culture vessel 12. In addition, the multilayer culture vessel 12 is communicated with the air filter 133 via the connecting portion 123. The air filter 133 is a filter blocking bacteria from being mixed into the multilayer culture vessel 12 while allowing air to pass therethrough, and is disposed between the inside and the outside of the multilayer culture vessel 12. In this embodiment, a clamp 132 is further disposed between the air filter 133 and the connecting portion 123 such that air can be taken into or discharged from the multilayer culture vessel 12 by controlling the clamp 132 to be opened and closed.

When performing the cell culture with the multilayer culture vessel 12, for example, the multilayer culture vessel 12 is rotated through about 90° counterclockwise to come into a state illustrated in Fig. 4(A) from a state illustrated in Fig. 3(C). Then, the clamps 131 and 132 are opened, and the culture solution containing the cells suspended therein is introduced into the multilayer culture vessel 12 via the liquid supply tube 13 in a state in which air inside the multilayer culture vessel 12 can be discharged to the outside through the air filter 133. Then, after closing the clamp 132 and rotating the multilayer culture vessel 12 through about 180° clockwise, the multilayer culture vessel 12 is returned to the upright state as illustrated in Fig. 4(B), whereby the culture solution is distributed to the individual trays 121 in the multilayer culture vessel 12. Then, the clamp 131 is closed and the cell culture is performed in the individual trays 121. During the cell culture, the clamp 132 is also kept closed state (depending on the type of the multilayer culture vessel 12, the culture may be performed with the clamp 132 being kept open). Fig. 4 is an explanatory view illustrating a manner of distributing the fluid material to the individual trays 121 in the multilayer culture vessel 12. More specifically, Fig. 4(A) is a schematic sectional view, like Fig. 3(C), of the multilayer culture vessel 12 taken along IIIC - IIIC in Fig. 3(A), and Fig. 4(B) is a schematic sectional view, like Fig. 3(B), of the multilayer culture vessel 12 taken along IIIB - IIIB in Fig. 3(A).

After the cell culture, a culture-solution discharging process of discharging the culture solution from the multilayer culture vessel 12, a releasing-solution (of trypsin, etc.) introducing process of introducing the releasing solution of trypsin, etc. into the multilayer culture vessel 12 to release the cells adhering to bottom surfaces of the individual trays 121 in the multilayer culture vessel 12, and a releasing-solution (of trypsin, etc.) recovering process of recovering the releasing solution of trypsin, etc., including the released cells, from the multilayer culture vessel 12 are performed. The above-mentioned processes for the fluid material (including the culture solution before the culture and the releasing solution such as a trypsin solution, the same shall apply to the following description) and the fluid (including the culture solution after the culture and the releasing solution of trypsin, etc., including the released cells) also need the operations of, for example, rotating or swinging the multilayer culture vessel 12 to introduce or discharge the fluid material, and the operations of opening and closing the clamps 131 and 132.

When the worker manually operates the multilayer culture vessel 12, the multilayer culture vessel 12 including the culture solution and the releasing solution of trypsin, etc. is heavy and the burden on the worker increases. Furthermore, the manual operation may lead to a possibility that a variation occurs in operations, or that the worker accidentally touches and damages the multilayer culture vessel 12, whereby the cell culture can no longer be continued in some cases. Moreover, because a certain large space is needed to rotate or swing the multilayer culture vessel 12, it has been usual to perform, outside a culture device (for example, a commercial culture-dedicated device), not only operations of attaching a liquid supply tube to the multilayer culture vessel 12, manipulating the multilayer culture vessel 12 so as to fill the culture solution into the multilayer culture vessel 12, and then placing the multilayer culture vessel 12 into the culture device, but also operations of taking out the multilayer culture vessel 12 from the culture device, exchanging the liquid supply tube to be attached to the multilayer culture vessel 12, and then manipulating the multilayer culture vessel 12. However, attaching the liquid supply tube to the multilayer culture vessel 12 and exchanging the liquid supply tube outside the culture device may accompany with a risk of causing the adverse effect due to temperature changes inside the multilayer culture vessel 12, etc.

To solve the above-described problems, the culture device 10 according to this embodiment is configured to be able to, after placing the multilayer culture vessel 12 in the internal space S of the housing 11, introduce the culture solution or the releasing solution of trypsin, etc. into the multilayer culture vessel 12 via the liquid supply tube 13 and to discharge the culture solution or the releasing solution of trypsin, etc. from the multilayer culture vessel 12 via the liquid supply tube 13 in a state in which the multilayer culture vessel 12 is placed in the internal space S without taking out the multilayer culture vessel 12 from the internal space S. More specifically, in the culture device 10 according to this embodiment, the multilayer culture vessel 12 is placed into the internal space S of the housing 11, and the liquid supply tube 13 is attached, within the internal space S, to the connecting portion 122 of the multilayer culture vessel 12, whereby the liquid supply tube 13 and the multilayer culture vessel 12 are communicated with each other. The liquid supply tube 13 is connected to the liquid supply tube 3a outside the housing 11 via the insertion portion 15 that is provided in a wall of the housing 11. There are no special limitations on a structure of the insertion portion 15 insofar as the inside and the outside of the housing 11 are connected to each other through the insertion portion 15. For example, the insertion portion 15 may be in the form of a hole with the same diameter as that of an outer periphery of the liquid supply tube 13. In that case, by inserting the liquid supply tube 13 through the insertion portion 15, the liquid supply tube 3a can be constituted by a portion of the liquid supply tube 13, the portion being positioned outside the housing 11.

The culture device 10 further includes the manipulator 14 to manipulate the multilayer culture vessel 12. The manipulator 14 includes a rotation unit 141 capable of holding the multilayer culture vessel 12, and a drive unit 142 driving and rotating the rotation unit 141. As illustrated in Fig. 2, the manipulator 14 drives the rotation unit 141 to rotate about two axes, namely rotation axes X1 and X2. As seen from Fig. 2, the rotation axis X1 is a rotation axis extending in an X-axis direction. Thus, the rotation unit 141 and the multilayer culture vessel 12 held by the rotation unit 141 can be both rotated in a roll direction R. The rotation axis X2 is a rotation axis extending in a Y-axis direction. Thus, the rotation unit 141 and the multilayer culture vessel 12 held by the rotation unit 141 can be both rotated in a pitch direction P. In a rotation operation, a rotation in the roll direction R is allowed over a range of ±180° or less. In this embodiment, the rotation unit 141 can be rotated in the roll direction R over a range of ±120°. A rotation in the pitch direction R is also allowed over a range of ±180° or less. In this embodiment, the rotation unit 141 can be rotated in the pitch direction R over a range of ±30°. Furthermore, in this embodiment, the drive unit 142 includes a first drive unit 1421 (an electric motor and/or an air cylinder) rotating the rotation unit 141 about the rotation axis X1 and a second drive unit 1422 (an electric motor and/or an air cylinder) rotating the rotation unit 141 about the rotation axis X2. Thus, the rotation unit 141 can be rotated about the two axes.

Moreover, the drive unit 142 of the manipulator 14 can perform a swing operation of reciprocally rotating the rotation unit 141 (and the multilayer culture vessel 12) about the rotation axis X1 or the rotation axis X2. For example, the drive unit 142 can perform the swing operation about the rotation axis X1 by reciprocally rotating the rotation unit 141 (and the multilayer culture vessel 12) over the range of ±120° in the roll direction R about the rotation axis X1. In addition, the drive unit 142 can perform the swing operation about the rotation axis X2 by reciprocally rotating the rotation unit 141 (and the multilayer culture vessel 12) in the pitch direction P over the range of ±30° about the rotation axis X2 in such a manner of first tilting an upper portion of the rotation unit 141 (and the multilayer culture vessel 12) forward (in an X-axis negative direction) and then tilting a lower portion of the rotation unit 141 (and the multilayer culture vessel 12) forward (in the X-axis negative direction).

The manipulator 14 further includes an opening/closing unit 143 opening and closing the clamp 131 or 132 for the liquid supply tube 13. Fig. 5 is an explanatory view illustrating an example of structures of the opening/closing unit 143 and the clamp 131. The opening/closing unit 143 may have a structure capable of, for example, pushing out a pressing member, such as a piston, toward the liquid supply tube 13 by a driver, such as an air cylinder, an electric cylinder, or a solenoid, and closing a flow path in the liquid supply tube 13 by tightly sandwiching the liquid supply tube 13 between the pushed-out piston and a stationary portion of the clamp 131. In such a case, the liquid supply tube 13 can be released from a sandwiched state to make the flow path in the liquid supply tube 13 open by pushing back the pressing member, such as the piston, which has been pushed out, toward the opposite side to the liquid supply tube 13 by a driver, such as an air cylinder, an electric cylinder, or a solenoid. The opening/closing unit 143 is not limited to the above-mentioned structure and can be formed by using a suitable known structure. A plurality of structures each operating in a similar manner to the combination of the clamp 131 and the opening/closing unit 143 may be disposed to serve as vents. In this embodiment, because the multilayer culture vessel 12 is rotated by the rotation unit 141 and the liquid supply tube 13 and the clamp 131 are also rotated concurrently, the opening/closing unit 143 is configured to be rotatable together with the rotation unit 141. The opening/closing unit 143 for opening and closing the clamp 132 can be constituted in a similar mechanism to that of opening/closing unit 143 for opening and closing the clamp 131.

The temperature adjustment unit 16 adjusts a temperature in the internal space S of the housing 11. The culture device 10 according to this embodiment includes the input unit 19 outside the housing 11, and the worker can set the temperature in the internal space S of the housing 11 by operating the input unit 19. The setting temperature input from the input unit 19 is sent to the temperature adjustment unit 16 via the control unit 18. When the temperature is set by the input unit 19, the temperature adjustment unit 16 adjusts the temperature in the internal space S such that the internal space S is held at the setting temperature.

The gas concentration adjustment unit 171 includes a gas concentration sensor for measuring a concentration of gas, such as carbon dioxide gas, inside the internal space S of the housing 11. Furthermore, the gas concentration adjustment unit 171 is connected to a gas supply unit disposed outside the housing 11 and introduces gas, such as carbon dioxide gas, into the internal space S in accordance with a measurement result of the gas concentration sensor, thereby adjusting the gas concentration inside the internal space S to a gas concentration that has been set by the worker via the input unit 19. Thus, the gas, such as the carbon dioxide gas, with an appropriate concentration can be supplied to the multilayer culture vessel 12. Instead of using the gas supply unit disposed outside the housing 11, the gas concentration adjustment unit 171 may include the gas supply unit therein (namely, may include the gas supply disposed inside the housing 11).

The pH adjustment unit 172 adjusts pH of the culture solution, etc. filled into the multilayer culture vessel 12. For example, the pH adjustment unit 172 includes a pH sensor for measuring pH of the fluid material filled into the multilayer culture vessel 12. Furthermore, the pH adjustment unit 172 is connected to a gas supply unit (not illustrated) disposed outside the housing 11 and can adjust, in accordance with a measurement result of the pH sensor, pH inside the multilayer culture vessel 12 to a value that has been set by the worker via the input unit 19.

The input unit 19 receives an instruction input by the worker and sends the input instruction to the control unit 18. The input unit 19 may be, for example, a button forming a switch, or a touch panel that also serves as a display.

The control unit 18 controls the operations of the manipulator 14, the temperature adjustment unit 16, and the pH adjustment unit 172 in accordance with instructions input by the worker via the input unit 19. Furthermore, the control unit 18 can control the operations of opening and closing the valves 2a to 2d, the operation of the opening/closing unit 143, the operations of the liquid supply pumps 20 and 70, and the operation of the centrifugal separator 60 in accordance with instructions input by the worker via the input unit 19.

The other components than the culture device 10 will be described below. The liquid supply pump 20 is connected, via the liquid supply tube 3a, to the liquid supply tube 13 and the multilayer culture vessel 12 that are both disposed in the culture device 10. The liquid supply pump 20 is further connected, via the liquid supply tubes 3b to 3e, to the containers 40 and 50 and the centrifugal separator 60. The liquid supply pump 20 delivers the culture solution or the releasing solution of trypsin, etc. from the container 40 or 50 to the multilayer culture vessel 12 and delivers the culture solution or the releasing solution of trypsin, etc. from the multilayer culture vessel 12 to the container 40 or 50. An amount of the culture solution or the releasing solution of trypsin, etc. delivered by the liquid supply pump 20 is measured by the flowmeter 30, and the liquid supply pump 20 can stop the delivery of the liquid automatically or manually in accordance with a measurement result of the flowmeter 30.

The culture solution containing the cells suspended therein is put into the container 40 to be ready for the cell culture. The liquid supply tube 3c is attached to the container 40, and the container 40 can be connected to or disconnected from the multilayer culture vessel 12 by opening or closing the valve 2a disposed near the container 40. When performing the cell culture, for example, the culture solution can be introduced into the multilayer culture vessel 12 from the container 40 by the liquid supply pump 20 with the valve 2a switched to an open state. After the cell culture, the culture solution can be delivered from the multilayer culture vessel 12 to the container 40 and recovered into the container 40 by the liquid supply pump 20 with the valve 2a switched to the open state.

The releasing solution of trypsin, etc. is put into the container 50 to be ready for cell recovery. The liquid supply tube 3d is attached to the container 50, and the container 50 can be connected to or disconnected from the multilayer culture vessel 12 by opening or closing the valve 2b disposed near the container 50. When performing the cell recovery, for example, the releasing solution of trypsin, etc. can be introduced into the multilayer culture vessel 12 from the container 50 by the liquid supply pump 20 with the valve 2b switched to an open state.

The centrifugal separator 60 performs centrifugal separation of the releasing solution of trypsin, etc., including the cultured cells after being subjected to a cell releasing process, thereby separating the cultured cells and the releasing solution of trypsin, etc. In this embodiment, the liquid supply tube 3e is attached to the centrifugal separator 60, and the centrifugal separator 60 can be connected to or disconnected from the multilayer culture vessel 12 by opening or closing the valve 2c disposed near the centrifugal separator 60. By switching the valve 2c to an open state after the cell releasing process, the releasing solution of trypsin, etc., including the cultured cells, can be introduced into the centrifugal separator 60 from the multilayer culture vessel 12 by the liquid supply pump 20. The operation of the centrifugal separator 60 may be automatically controlled by the control unit 18. Instead, the centrifugal separator 60 may be manually operated by the worker.

In this embodiment, the centrifugal separator 60 is connected to the liquid supply pump 70 and the container 80 via, respectively, the liquid supply tubes 3f and 3g. After performing the centrifugal separation of the releasing solution of trypsin, etc. and washing in the centrifugal separator 60, the cells having been separated and recovered from the releasing solution of trypsin, etc. in the centrifugal separator can be delivered into the container 80 by the liquid supply pump 70 with the valve 2c switched to a closed state and the valve 2d switched to an open state.

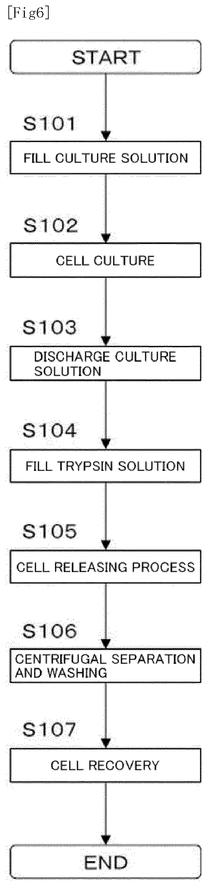

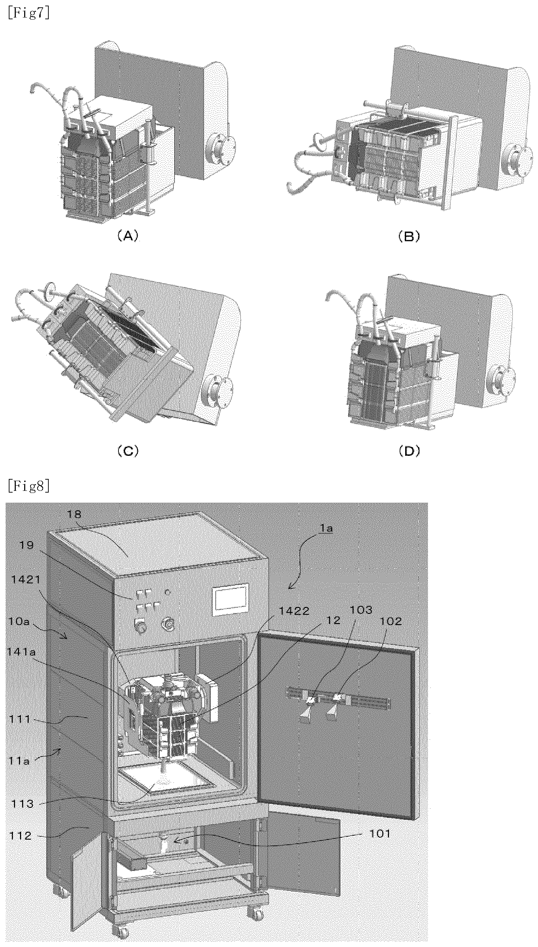

A cell culture method using the culture system 1 according to the first embodiment will be described below. Fig. 6 is a flowchart illustrating the cell culture method according to the first embodiment. Fig. 7 is an explanatory view illustrating an operation of rotating the multilayer culture vessel 12 according to the first embodiment. In Fig. 7, for convenience of explanation, only the multilayer culture vessel 12 and the manipulator 14 are illustrated, and the other components are omitted.

First, as illustrated in Fig. 6, a process of introducing the culture solution into the multilayer culture vessel 12 is performed in step S101. More specifically, at the beginning, the worker prepares the container 40 in which the culture solution containing the cells suspended therein is put, attaches the liquid supply tube 3c to the container 40, and opens the valve 2a. Then, the worker operates the input unit 19 to open the clamp 131 for the liquid supply tube 13 by the opening/closing unit 143, thus communicating the container 40 and the multilayer culture vessel 12 with each other. The worker further opens the clamp 132 to allow air inside the multilayer culture vessel 12 to be discharged through the air filter 133. Thereafter, the worker operates the input unit 19 and instructs the manipulator 14 of the culture device 10 to perform a rotation operation of rotating, from the state illustrated in Fig. 7(A) or illustrated in Fig. 3(C), the multilayer culture vessel 12 through about 90° such that the connecting portion 122 of the multilayer culture vessel 12 is positioned on a lower side and the connecting portion 123 is positioned on an upper side as illustrated in Fig. 7(B) or 4(A). In addition, the worker actuates the liquid supply pump 20 to introduce the culture solution into the multilayer culture vessel 12 from the container 40. An amount of the culture solution introduced into the multilayer culture vessel 12 from the container 40 is measured by the flowmeter 30 and, when delivery of the culture solution from the liquid supply pump 20 comes to an end, the operation of the liquid supply pump 20 is stopped. Upon the end of delivery of the culture solution, the clamp 132 is closed to protect the air filter 133 from the culture solution. After the culture solution has been introduced into the multilayer culture vessel 12, the worker operates the input unit 19 and instructs the manipulator 14 of the culture device 10 to perform rotation operations of rotating, as illustrated in Fig. 7(C), the multilayer culture vessel 12 through about 180° such that the connecting portion 122 of the multilayer culture vessel 12 is positioned on the upper side and the connecting portion 123 is positioned on the lower side, and then further rotating the multilayer culture vessel 12 to be positioned upright as illustrated in Fig. 7(D). Thus, as illustrated in Fig. 4(B), the culture solution containing the cells suspended therein can be distributed to the individual trays 121. Then, operations of closing the valve 2a and the clamp 131 are performed.

In step S102, the cell culture is performed. For example, the worker can previously set the temperature in the internal space S of the housing 11 via the input unit 19, and the temperature adjustment unit 16 adjusts the temperature in the internal space S of the housing 11 to the temperature set by the worker. Then, the worker can perform the cell culture by progressing static culture or shaking culture in the internal space S of the housing 11 for a predetermined time with the multilayer culture vessel 12 that includes the culture solution containing the cells suspended therein.

In step S103, the culture solution is recovered. For example, the worker operates the input unit 19 to instruct the manipulator 14 to open the clamps 131 and 132 and to rotate the multilayer culture vessel 12 as illustrated in Fig. 7(B) or 4(A). Furthermore, the worker opens the valve 2a to communicate the container 40 and the multilayer culture vessel 12 with each other and then operates the liquid supply pump 20 to deliver the culture solution from the multilayer culture vessel 12 to the container 40. As a result, the culture solution is recovered to the container 40, and the cultured cells remain in the multilayer culture vessel 12 while adhering to the trays 121.

In step S104, a process of introducing the releasing solution of trypsin, etc. into the multilayer culture vessel 12 is performed to release the cells adhering to the trays 121. More specifically, while keeping the clamps 131 and 132 open, the worker closes the valve 2a and opens the valve 2b near the container 50 in which the releasing solution of trypsin, etc. is put, thus communicating the container 50 and the multilayer culture vessel 12 with each other. Then, the worker operates the liquid supply pump 20 to introduce the releasing solution of trypsin, etc. into the multilayer culture vessel 12 via the liquid supply tubes 3d, 3b and 3a and the liquid supply tube 13. When introducing the releasing solution of trypsin, etc. into the multilayer culture vessel 12, the worker operates the input unit 19 to perform the rotation operation of rotating the multilayer culture vessel 12, as illustrated in Figs7(A) to 7(D), by causing the drive unit 142 of the manipulator 14 to rotate the rotation unit 141 as in step S101.

In step S105, the cell releasing process is performed. For example, the worker can instruct the manipulator 14 to swing the multilayer culture vessel 12 by operating the input unit 19 and inputting the instruction. Furthermore, a manipulation program for the cell releasing process is programmed to reciprocally swing the multilayer culture vessel 12 in a first direction (for example, a rightward direction) and a second direction (for example, a leftward direction) about the first rotation axis X1 or the second rotation axis X2 by the rotation unit 141. In addition, the manipulation program includes a stop mode of stopping the movement of the multilayer culture vessel 12 for a designated time at the switch timing from the rotation operation in the first direction to the rotation operation in the second direction and at the switch timing from the rotation operation in the second direction to the rotation operation in the first direction. With the stop mode of stopping the swing operation at the switch timing of the direction of the rotation operation for the designated time, even when the swing operation is performed at a faster speed than the movement of the liquid within the vessel, it is possible to reliably apply shearing force to the cells adhering to the trays 121 with the liquid within the vessel and to promote the releasing of the cells from the trays 121. Although swinging the vessel at a high speed is important to effectively perform the cell releasing process, the presence of the stop mode can eliminate the problem of a delay (time lag) in the movement of the liquid, which is caused when the vessel is swung at the high speed.

In step S106, a centrifugal separation process is performed. More specifically, the worker operates the input unit 19 to instruct the manipulator 14 to rotate the multilayer culture vessel 12 such that the connecting portion 122 is positioned on the lower side, and to open the clamps 131 and 132. Furthermore, the worker operates the input unit 19 to open the valve 2c disposed near the centrifugal separator 60 and to communicate the multilayer culture vessel 12 and the centrifugal separator 60 with each other. Then, the worker operates the liquid supply pump 20 to introduce the releasing solution of trypsin, etc., including the released cells, into the centrifugal separator 60 via the liquid supply tube 13 and the liquid supply tubes 3a, 3b and 3e. In addition, the worker operates the centrifugal separator 60 to perform centrifugal separation of the releasing solution of trypsin, etc., including the cells, thereby separating the cultured cells and the releasing solution of trypsin, etc.

In step S107, a device constituted by a service tank, a continuous centrifugal separator, a culture solution supply tank, a cell recovery bag, a waste recovery bag, a pump, and so on can be used as the centrifugal separator 60. A process of recovering the cultured cells is performed. For example, the worker can recover the cultured cells that have precipitated with the operation of the centrifugal separator 60. After closing the valve 2c and opening the valve 2d to communicate the centrifugal separator 60 and the container 80 with each other, the worker operates the liquid supply pump 70 to deliver the culture solution containing the cells separated and suspended therein from the centrifugal separator 60 to the container 80, thus recovering the cultured cells.

As described above, the culture system 1 according to the first embodiment includes the housing 11 with the internal space S in which the multilayer culture vessel 12 including the plurality of the trays 121 therein is placed, the temperature adjustment unit 16 adjusting the temperature in the internal space S, and the manipulator 14 manipulating the multilayer culture vessel 12 while the multilayer culture vessel 12 is kept in a state placed within the internal space S. The multilayer culture vessel 12 is communicated with the liquid supply tube 13 such that the culture solution or the releasing solution of trypsin, etc. can be introduced into the multilayer culture vessel 12 from the outside of the housing 11 via the liquid supply tube 13, or that the culture solution can be discharged from the multilayer culture vessel 12 to the outside of the housing 11 via the liquid supply tube 13. With the above configuration, since the manipulation of the multilayer culture vessel 12 and the cell culture can be performed in sequence in the same space, labor and time required for the worker to take out the multilayer culture vessel 12 from the incubator and to place the multilayer culture vessel 12 into the incubator for each manipulation of the multilayer culture vessel 12 can be eliminated, and the burden on the worker can be reduced. Moreover, since there is no need of exchanging, for example, the liquid supply tube 13 outside the incubator, temperature changes causing the adverse effect on the cell culture can be effectively prevented.

The above-mentioned series of operations can be automatically progressed step by step even without the presence of the worker.

In addition, by providing a plurality of the culture systems 1 according to this embodiment, the cell culture, the cell releasing, and the recovery of the cultured cells can be performed by using a plurality of the multilayer culture vessels 12, and the burden on the worker can be further reduced.

### <Second Embodiment>

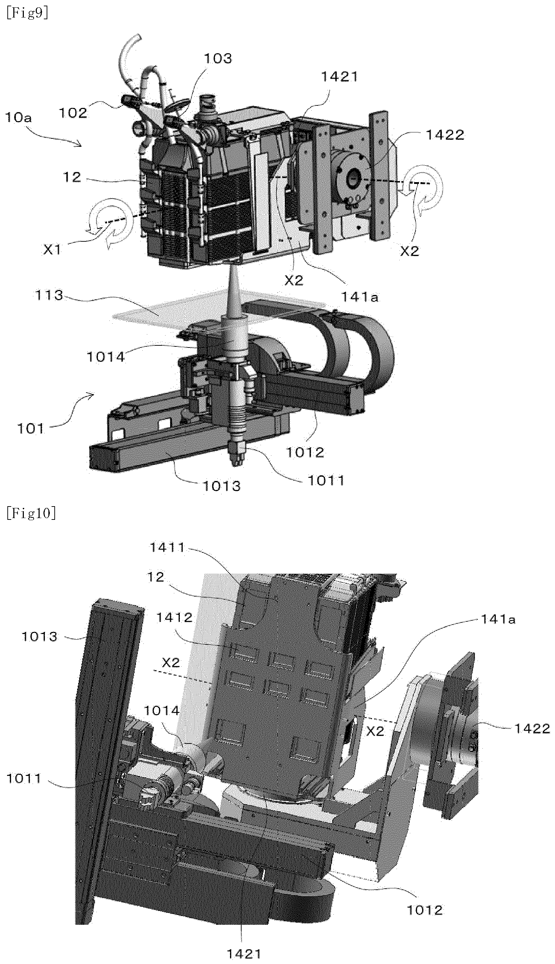

A culture system 1a according to a second embodiment will be described below. Fig. 8 is a perspective view of the culture system 1a according to the second embodiment, and Fig. 9 is a perspective view illustrating a structure inside a housing 11a of the culture system 1a according to the second embodiment. The culture system 1a according to the second embodiment has a similar configuration to the culture system 1 according to the first embodiment except for including an observation device 101, two liquid level sensors 102 and 103, and a rotation unit 141a constituted such that a rotation axis X2 of the rotation unit 141a passes the multilayer culture vessel 12. The culture system 1a according to the second embodiment is operated in a similar manner to the culture system 1 according to the first embodiment. For convenience of explanation, regarding the observation device 101 and the liquid level sensors 102 and 103, their visual fields are illustrated in Figs. 8 and 9 (this point is similarly applied to Fig. 10 described later).

More specifically, in the culture system 1a according to the second embodiment, as illustrated in Fig. 8, a space S inside the housing 11a is divided by a partition plate 113 into two parts, namely an upper accommodation portion 111 and a lower accommodation portion 112. A culture device 10a including the multilayer culture vessel 12 is placed in the upper accommodation portion 111, and the observation device 101 is placed in the lower accommodation portion 112. A part or the whole of the partition plate 113 is made of a transparent material (transparent resin or glass) such that the interior of the multilayer culture vessel 12 placed in the upper accommodation portion 111 can be observed at a position of the transparent material by the observation device 101 placed in the lower accommodation portion 112.

As illustrated in Fig. 9, the observation device 101 includes a camera 1011, a first camera driver 1012, a second camera driver 1013, and a lens 1014. The camera 1011 is, for example, a CCD camera or a CMOS camera and is installed under the multilayer culture vessel 12 to take an image of an inner bottom side of the tray 121 in the multilayer culture vessel 12 from below the multilayer culture vessel 12 through the lens 1014. The image (including a moving image) taken by the observation device 101 is sent to a display device (not illustrated) and is displayed on the display device to be viewed by the worker. In this embodiment, the multilayer culture vessel 12 is held by a rotation unit 141a, and a plurality of cutouts 1412 are formed in a bottom surface 1411 of the rotation unit 141a as illustrated in Fig. 10. The camera 1011 can take the image of the inner bottom side of the tray 121 in the multilayer culture vessel 12 through any one of the cutouts 1412. Fig. 10 is a perspective view illustrating the structure inside the housing 11a of the culture system 1a according to the second embodiment when viewed from below. In other words, Fig. 10 illustrates the culture system 1a when viewed from an observation position at which the cells in the multilayer culture vessel 12 are observed by the observation device 101.

In this embodiment, the camera 1011 is coupled to the first camera driver 1012 such that the camera 1011 can be linearly moved in a predetermined first direction. Furthermore, the second camera driver 1013 is coupled to the first camera driver 1012 such that the camera 1011 can be linearly moved together with the first camera driver 1012 in a second direction perpendicular to the first direction. Thus, the camera 1011 can be freely moved in two-dimensional directions by the first camera driver 1012 and the second camera driver 1013. Therefore, the worker can observe a culture state through the cutout 1412 at a desired position. A two-dimensional position of the camera 1011 can be selected by the worker inputting an instruction via the input unit 19. An illumination device (not illustrated) may be installed on a side opposite to the camera 1011 with the multilayer culture vessel 12 interposed therebetween. In such a case, by aligning an optical axis of the illumination device and an optical axis of the camera 1011 with each other, illuminance on the multilayer culture vessel 12 can be increased, and an image representing a state of the cultured cells can be taken with appropriate brightness.

As illustrated in Figs. 8 and 9, the culture system 1a according to the second embodiment further includes a first liquid level sensor 102 and a second liquid level sensor 103. The first liquid level sensor 102 and the second liquid level sensor 103 are each a sensor that is used to, when the fluid material, such as the culture solution or the releasing solution of trypsin, etc., is introduced into the multilayer culture vessel 12, determine whether the fluid material has been introduced into the multilayer culture vessel 12 until reaching an appropriate amount (predetermined amount necessary for the culture). Each of the first liquid level sensor 102 and the second liquid level sensor 103 includes a camera, such as a CCD or CMOS camera, detects in accordance with the image (including the moving image) taken by the camera that the appropriate amount of the fluid material has been introduced into the multilayer culture vessel 12, and outputs, to the control unit 18, a signal for stopping the supply of the fluid material to the multilayer culture vessel 12.

More specifically, the first liquid level sensor 102 and the second liquid level sensor 103 are each installed to be fixed to an inner wall of the housing 11a and to take an image of a liquid level of the fluid material that has been introduced into the multilayer culture vessel 12. When introducing the fluid material into the multilayer culture vessel 12, the fluid material is introduced into the multilayer culture vessel 12 in a state in which the multilayer culture vessel 12 is tilted through about 90° by the rotation unit 141a. While the fluid material is introduced into the multilayer culture vessel 12 as described above, the second liquid level sensor 103 takes an image of the vicinity of a second water level a little lower than a first water level at which the amount of the fluid material introduced into the multilayer culture vessel 12 is appropriate. Then, when the liquid level of the fluid material has reached the second water level, the second liquid level sensor 103 detects that the culture solution or the releasing solution of trypsin, etc. has been introduced in an amount close to the appropriate amount, and sends a signal to the control unit 18. Upon receiving the signal from the second liquid level sensor 103, the control unit 18 determines that the amount of the introduced fluid material has reached a value close to the appropriate amount, and controls the operation of the liquid supply pump 20 to reduce an inflow amount of the fluid material.

While the fluid material is introduced into the multilayer culture vessel 12, the first liquid level sensor 102 takes an image of the vicinity of the first water level at which the amount of the introduced fluid material is appropriate. Then, when the liquid level of the fluid material has reached the first water level at which the amount of the introduced fluid material is appropriate, the first liquid level sensor 102 detects that the amount of the introduced fluid material is appropriate, and sends a signal to the control unit 18. Upon receiving the signal from the first liquid level sensor 102, the control unit 18 determines that the amount of the introduced fluid material has reached an appropriate value, and controls the operations of opening and closing the valves 2a to 2d, the operation of the opening/closing unit 143, and the operation of the liquid supply pump 20 to stop the inflow of the fluid material.

Furthermore, in the second embodiment, the structure of the rotation unit 141a is different from that of the rotation unit 141 in the first embodiment. More specifically, in the rotation unit 141 in the first embodiment, as illustrated in Fig. 2, the rotation axis X2 about which the multilayer culture vessel 12 is rotated in the pitch direction P passes the rotation unit 141, and the multilayer culture vessel 12 is rotated in the pitch direction P about the rotation axis X2.

On the other hand, in the second embodiment, as illustrated in Figs. 9 and 10, the rotation unit 141a includes a first drive unit 1421 rotating the multilayer culture vessel 12 in the roll direction R and a second drive unit 1422 rotating the multilayer culture vessel 12 in the pitch direction P. The second drive unit 1422 is installed such that its rotation axis X2 passes the multilayer culture vessel 12. Stated in another way, as illustrated in Figs. 9 and 10, the second drive unit 1422 is disposed on a lateral side of the multilayer culture vessel 12, and hence the multilayer culture vessel 12 can be rotated in the pitch direction P about the rotation axis X2 passing the multilayer culture vessel 12. Therefore, the second drive unit 1422 in the second embodiment can rotate the multilayer culture vessel 12 over a wider range of angle. Thus, in the culture system 1 according to the first embodiment, the multilayer culture vessel 12 is rotatable by the second drive unit 1422 only within the range of ±30° in the pitch direction P about the rotation axis X2. In the culture system 1a according to the second embodiment, however, the multilayer culture vessel 12 is rotatable by the second drive unit 1422 through 90° or more in the pitch direction P (specifically, in the clockwise pitch direction P in Fig. 9) about the rotation axis X2. As a result, the multilayer culture vessel 12 can be swung in a state in which the multilayer culture vessel 12 is rotated through 90°. The first drive unit 1421 rotates the multilayer culture vessel 12 in the roll direction R, as in the first embodiment, about the rotation axis X1 passing the multilayer culture vessel 12.

As described above, the culture system 1a according to the second embodiment includes the observation device 101 disposed under the multilayer culture vessel 12 and taking the image of the inner side of the tray 121 from the bottom side in the multilayer culture vessel 12. Therefore, the worker can appropriately grasp a cell culture state in the multilayer culture vessel 12 without taking out the multilayer culture vessel 12 from the housing 11a. Furthermore, in this embodiment, the plurality of the cutouts 1412 are formed in the bottom surface 1411 of the rotation unit 141a, and a cell state on the inner bottom side of the tray 121 in the multilayer culture vessel 12 can be observed by the observation device 101 through any one of the cutouts 1412.

The culture system 1a according to the second embodiment further includes the second liquid level sensor 103 detecting that the fluid material, such as the culture solution, has been introduced in an amount close to the appropriate amount, and the first liquid level sensor 102 detecting that the amount of the introduced fluid material has reached the appropriate value. In the culture system 1a according to the second embodiment, therefore, the supply of the fluid material can be automatically stopped when the fluid material has been introduced to the appropriate amount in the multilayer culture vessel 12.

Moreover, in the culture system 1a according to the second embodiment, the second drive unit 1422 is installed such that the rotation axis X2 of the second drive unit 1422 rotating the multilayer culture vessel 12 in the pitch direction P passes the multilayer culture vessel 12. In the first embodiment, because the rotation axis X2 for the multilayer culture vessel 12 in the pitch direction P and the multilayer culture vessel 12 are away from each other, a space necessary for the rotation of the multilayer culture vessel 12 in the pitch direction P is increased corresponding to a distance between the rotation axis X2 and the multilayer culture vessel 12, and an overall size of the culture system 1 is also increased. In the second embodiment, however, because the distance between the rotation axis X2 about which the multilayer culture vessel 12 is rotated in the pitch direction P and the multilayer culture vessel 12 is short, the space necessary for the rotation of the multilayer culture vessel 12 can be reduced and hence the overall size of the culture system 1 can be reduced corresponding to the reduction of the space.

The preferred embodiments of the present invention have been described above, but the technical scope of the present invention is not limited to the matters described in the above embodiments. The above embodiments can be variously modified and improved, and the modified and improved embodiments also fall within the technical scope of the present invention.

In the above embodiments, the culture device 10 and the culture system 1 including the culture device 10 have been described, by way of example, as the embodiments of the culture device and the culture system according to the present invention. However, a device including the manipulator 14 and used in the culture device 10, as illustrated in Fig. 7, can be further provided as the multilayer culture vessel manipulation device according to the present invention.

In the above embodiments, the culture device 10 has been described, by way of example, as including the temperature adjustment unit 16, the gas concentration adjustment unit 171, and the pH adjustment unit 172. However, the present invention is not limited to such a configuration, and the culture device 10 may include one or two among the temperature adjustment unit 16, the gas concentration adjustment unit 171, and the pH adjustment unit 172.

The above embodiments have been described, by way of example, as having the following configuration. As illustrated in Fig. 1, the valves 2a to 2c are disposed on separate liquid supply tubes (the liquid supply tubes 3c to 3e) communicating with the containers 40, 50 and 80, respectively, and communication states between the separate liquid supply tubes (the liquid supply tubes 3c to 3g) and common liquid supply tubes (the liquid supply tubes 3a, 3b and 13) are controlled to be closed or opened by controlling the valves 2a to 2c. However, the present invention is not limited to such a configuration. In another example, valves may be disposed at junctions between the separate liquid supply tubes (the liquid supply tubes 3c to 3g) and the common liquid supply tubes (the liquid supply tubes 3a and 3b), and the communication states between the separate liquid supply tubes (the liquid supply tubes 3c to 3g) and the common liquid supply tubes (the liquid supply tubes 3a, 3b and 13) may be controlled by controlling those valves.

The above embodiments have been described, by way of example, as using camera sensors as the liquid level sensors 102 and 103. However, the present invention is not limited to such a case, and an ultrasonic, capacitive, or pressure-type liquid level sensor may also be used. Among those sensors, the capacitive liquid level sensor is preferably used. In the case of using the ultrasonic or capacitive liquid level sensor, by attaching the liquid level sensor to a predetermined position (namely, a position at which the sensor can detect the first water level or the second water level) in the rotation unit 141, whether the fluid material has reached the first water level or the second water level can be determined even when the multilayer culture vessel 12 is exchanged. Furthermore, for convenience of explanation, the above embodiments have been described, by way of example, as using the liquid level sensors 102 and 103 to determine whether the culture solution or the releasing solution of trypsin, etc. has reached the first water level or the second water level. However, the configuration may be modified to make the culture solution and the releasing solution of trypsin, etc. capable of being injected up to water levels different from each other. In such a case, for example, the water level of the culture solution may be monitored by the first liquid level sensor 102 and the second liquid level sensor 103, while the water level of the releasing solution of trypsin, etc. may be monitored by a third liquid level sensor and a fourth liquid level sensor that are different from the first liquid level sensor 102 and the second liquid level sensor 103. In other words, the fourth liquid level sensor may be used to determine whether the releasing solution of trypsin, etc. has reached a fourth water level a little lower than a third water level at which an amount of the releasing solution of trypsin, etc. injected into the multilayer culture vessel 12 is appropriate, and the third liquid level sensor may be used to determine whether the releasing solution of trypsin, etc. has reached the third water level. In addition, another liquid level sensor may be disposed to determine that the multilayer culture vessel 12 has become empty when the fluid material is recovered from the multilayer culture vessel 12.

### List of Reference Signs

- 1, 1a...: culture system
- 10, 10a...: culture device
- 11, 11a...: housing
- 111...: upper accommodation portion
- 112...: lower accommodation portion
- 113...: partition plate
- 12...: multilayer culture vessel
- 121...: tray
- 122, 123...: connecting portion 123... liquid supply tube
- 131, 132...: clamp
- 133...: air filter
- 14...: manipulator
- 141, 141a...: rotation unit
- 1411...: bottom surface
- 1412...: cutout
- 142...: drive unit
- 1421...: first drive unit
- 1422...: second drive unit
- 143...: opening/closing unit
- 15...: insertion portion
- 16...: temperature adjustment unit
- 171...: gas concentration adjustment unit
- 172...: pH adjustment unit
- 18...: control unit
- 19...: input unit
- 101...: observation device
- 1011...: camera
- 1012...: first camera driver
- 1013...: second camera driver
- 1014...: lens
- 102...: first liquid level sensor
- 103...: second liquid level sensor
- 20...: liquid supply pump
- 30...: flowmeter
- 40...: container (for supply and recovery of culture solution)
- 50...: container (for supply of releasing solution of trypsin etc.)
- 60...: centrifugal separator
- 70...: liquid supply pump
- 80...: container (for cell recovery)
- 2a to 2d...: valve
- 3a to 3g...: liquid supply pipe

## Claims

1. A culture system comprising:
a housing with an internal space in which a multilayer culture vessel including a plurality of trays therein is placed; and
a manipulator manipulating the multilayer culture vessel while the multilayer culture vessel is kept in a state placed within the internal space,
wherein the multilayer culture vessel is communicated with a liquid supply tube such that a fluid material can be introduced into the multilayer culture vessel from an outside of the housing via the liquid supply tube, or that a fluid can be discharged from the multilayer culture vessel to the outside of the housing via the liquid supply tube.

2. The culture system according to claim 1, wherein the manipulator includes a rotation unit rotating or swinging the multilayer culture vessel.

3. The culture system according to claim 1 or 2, wherein the manipulator includes an opening/closing unit opening and closing a communication path between the liquid supply tube and the multilayer culture vessel.

4. The culture system according to any one of claims 1 to 3, further comprising an air filter between an inside and an outside of the multilayer culture vessel,
wherein the manipulator includes an opening/closing unit opening and closing a communication path between the air filter and the multilayer culture vessel.

5. The culture system according to any one of claims 1 to 4, wherein the housing includes an insertion portion at which the liquid supply tube penetrates through the housing, and via both the insertion portion and the liquid supply tube, the fluid material is introduced into the multilayer culture vessel from the outside of the housing, or the fluid is discharged from the multilayer culture vessel to the outside of the housing.

6. The culture system according to any one of claims 1 to 5, wherein the liquid supply tube may be communicated with a plurality of containers or devices present outside the housing in a switchable manner.

7. The culture system according to claim 6, wherein a plurality of separate liquid supply tubes in communication with the plurality of containers in a one-to-one relation are communicated with a common liquid supply tube that is communicated with the multilayer culture vessel, valves are disposed on the separate liquid supply tubes or at junctions between the separate liquid supply tubes and the common liquid supply tube, and communication states between the separate liquid supply tubes and the common liquid supply tube are controllable with control of the valves.

8. The culture system according to any one of claims 1 to 7, further comprising an observation device disposed under the multilayer culture vessel and taking an image of an inside of the tray in the multilayer culture vessel.

9. A culture device comprising:
a housing with an internal space in which a multilayer culture vessel including a plurality of trays therein is placed; and
a manipulator manipulating the multilayer culture vessel while the multilayer culture vessel is kept in a state placed within the internal space,
wherein the multilayer culture vessel is communicated with a liquid supply tube such that a fluid material can be introduced into the multilayer culture vessel from an outside of the housing via the liquid supply tube, or that a fluid can be discharged from the multilayer culture vessel to the outside of the housing via the liquid supply tube.

10. The culture device according to claim 9, wherein the manipulator includes a rotation unit rotating or swinging the multilayer culture vessel.

11. The culture device according to claim 9 or 10, wherein the manipulator includes an opening/closing unit opening and closing a communication path between the liquid supply tube and the multilayer culture vessel.

12. A multilayer culture vessel manipulation device placed within a housing and including a manipulator that manipulates a multilayer culture vessel including a plurality of trays therein,
wherein the multilayer culture vessel is communicated with the liquid supply tube inside the housing,
the manipulator is able to manipulate the multilayer culture vessel while the multilayer culture vessel is kept in a state placed within the housing, and
with the manipulator manipulating the multilayer culture vessel, a fluid material can be introduced into the multilayer culture vessel from an outside of the housing via the liquid supply tube, or a fluid can be discharged from the multilayer culture vessel to the outside of the housing via the liquid supply tube.

13. The multilayer culture vessel manipulation device according to claim 12, wherein the manipulator includes a rotation unit rotating or swinging the multilayer culture vessel.

14. The multilayer culture vessel manipulation device according to claim 13, wherein the rotation unit has a first rotation axis and a second rotation axis each serving as a rotation axis about which the multilayer culture vessel is rotated or swung, and
the rotation unit is constituted such that the first rotation axis and the second rotation axis are each a rotation axis passing the multilayer culture vessel.

15. The multilayer culture vessel manipulation device according to any one of claims 12 to 14, wherein the manipulator includes an opening/closing unit opening and closing a communication path between the liquid supply tube and the multilayer culture vessel.

16. The multilayer culture vessel manipulation device according to any one of claims 12 to 15, further comprising a liquid level sensor detecting a liquid level of the fluid material in the multilayer culture vessel when the fluid material is introduced into the multilayer culture vessel from the outside of the housing via the liquid supply tube.

17. The multilayer culture vessel manipulation device according to claim 16, wherein the liquid level sensor includes a first liquid level sensor detecting whether the fluid material has reached a first water level indicating that a predetermined amount of the fluid material has been introduced into the multilayer culture vessel, or a second liquid level sensor detecting whether the fluid material has reached a second water level being lower than the first water level and indicating that the fluid material has been introduced in an amount close to the predetermined amount.
